# EUROPEAN PATENT APPLICATION

(11) **EP 4 368 149 A1**
(43) Date of publication of application: **15.05.2024**
(21) Application number: 21948981.2
(22) Date of filing: 20.08.2021
(51) Int. Cl.: A61F 2/24, A61F 2/95, A61F 2/966

(54) **IMPLANT CONVEYING HANDLE, IMPLANT SYSTEM, IMPLANT CONVEYING SYSTEM, AND WORKING METHOD THEREOF**

(30) Priority: 05.07.2021 CN 202110756496
(71) Applicant: Shanghai Trulive Medtech Co., Ltd., Shanghai 201206 (CN); Jiangsu Trulive Medtech Co., Ltd., Jiangsu 330038 (CN)
(72) Inventor: ZHANG, Wei, Shanghai 201206 (CN); XU, Haoran, Shanghai 201206 (CN); WEN, Jing, Shanghai 201206 (CN); SUN, Yunyan, Shanghai 201206 (CN)
(74) Representative: Nordmeyer, Philipp Werner
(86) International application number: PCT/CN2021/113830
(87) International publication number: WO 2023/279492

(57) **Abstract**

The present invention provides an implant delivery handle, an implant system, a delivery system and a method of operation thereof. The implant delivery handle includes two conveyance sub-systems both oriented along an axial direction of a catheter assembly, in which a distal one is coupled to a proximal portion of an outer tube in the catheter assembly, and a proximal one is coupled to a proximal portion of an inner tube in the catheter assembly. Each of the conveyance sub-systems comprises a synchronous conveying device and a ratchet device fixed to the synchronous conveying device. The ratchet device is configured to enable safe self-locking, transmission and guidance of the outer or inner tube through the synchronous conveying device. In this way, both the inner and outer tubes can be locked in one direction, avoiding opposite movement of the inner or outer tube caused by incorrect operation of an operator. Therefore, safe self-locking of the implant delivery handle is achieved, and uncontrolled displacement of the inner or outer tube can be avoided.

## Description

### TECHNICAL FIELD

The present invention relates to the technical field of medical devices and, in particular, to an implant delivery handle, an implant system, an implant delivery system and a method of operation thereof.

### BACKGROUND

Heart valves are openable and closeable flap-like structures in the organs of humans and some animals. The human heart has four valves: the aortic valve connecting the left ventricle to the aorta; the pulmonary valve connecting the right ventricle to the pulmonary artery; the mitral valve connecting the left atrium to the left ventricle; and the tricuspid valve connecting the right atrium to the right ventricle. All these valves function like one-way valves which allow blood flow in the forward direction but prevent its backward flow.

With the development of social economy and the aging of population, the incidence of valvular heart disease has increased significantly. Studies have shown that this figure has reached as high as 13.3% among those 75 years or older. At present, traditional surgical treatment remains the first choice for patients with severe valvular disease. However, for those with advanced ages, complications in multiple organs, a history of thoracotomy or poor heart functions, the traditional surgical approach is associated with high risk and high mortality or even precludes some patients.

Interventional valve implantation is a brand new minimally invasive valve replacement technique developed abroad in recent years, which involves loading valve prosthesis in a delivery system and delivering it into a human body through a catheter. The prosthesis can functionally replace the patient's dysfunctional native valve and improve his/her cardiac condition. This technique is able to treat valvular disease, without surgically opening the chest or stopping the heartbeat, which may cause significant trauma to the patient.

The structure of the human heart is very complex. In particular, the structure of the mitral valve is more complex even than that of the aortic valve because the mitral annulus has an irregular shape and there are many chordae tendineae in the ventricular chamber, which pose great challenges to the implantation and positioning of a prosthetic valve. For transcatheter valve replacement (including transcatheter aortic valve replacement (TAVI), transcatheter mitral valve replacement (TMVR), etc.), accurate valve stent placement is one of the key factors for surgical success. Accordingly, delivery systems for this purpose are required to allow operation, in particular, guidance, transmission, self-locking and the like, to be performed in a precise manner. Non-precise operation of such delivery systems may lead to operator mistakes during surgical procedures, inaccurate stent placement, or in severe cases, paravalvular leakage, regurgitation or other serious consequences.

### SUMMARY OF THE INVENTION

It is an object of the present invention to provide an implant delivery handle capable of precise operation, which enable guidance, power transmission, self-locking and other operation of an implant delivery system.

It is another object of the present invention to provide an implant system, a delivery system and a method of operation thereof, which enable guidance, transmission, self-locking and other operation of inner and outer tubes, as well as precise operation of the delivery system.

To this end, the present invention provides an implant delivery handle comprising two conveyance sub-systems both oriented along an axial direction of a catheter assembly, in which a distal one is coupled to a proximal portion of an outer tube in the catheter assembly, and a proximal one is coupled to a proximal portion of an inner tube in the catheter assembly,
wherein each of the conveyance sub-systems comprises a synchronous conveying device and a ratchet device fixed to the synchronous conveying device, the ratchet device configured to enable safe self-locking, transmission and guidance of the outer or inner tube through the synchronous conveying device.

Optionally, each of the conveyance sub-systems may further comprise a retention mechanism and a handwheel, the retention mechanism coupled both to the synchronous conveying device and to the outer or inner tube, the handwheel coupled to the synchronous conveying device and configured to drive, through the synchronous conveying device and the retention mechanism, the outer or inner tube to move axially.

Additionally, the ratchet device may comprise a ratchet wheel, a ratchet slider, a slider pin and a slider base,
the slider base configured to accommodate the ratchet slider;
the ratchet slider having one end resiliently secured to the slider base and a further end disposed near the ratchet wheel, the ratchet slider configured to control whether the ratchet wheel is rotated and a direction of rotation of the ratchet wheel;
the ratchet wheel fixed to the synchronous conveying device and configured to cooperate with the ratchet slider to control whether the outer or inner tube to move axially and a direction of axial movement of the outer or inner tube,
the slider pin coupled to the one end of the ratchet slider and configured to drive the ratchet slider to move.

Additionally, the synchronous conveying device may comprise a driving pulley, a driven pulley and a synchronous conveying belt, the synchronous conveying belt provided on both the driving pulley and the driven pulley, the driving pulley disposed coaxially with the handwheel so as to rotate therewith, the driving pulley configured to drive the driven pulley to rotate in a direction in which the driving pulley rotates and cause the synchronous conveying belt to move in an axial direction of the outer tube.

Additionally, the ratchet wheel may be disposed coaxially with the handwheel and located between the driving pulley and the handwheel, wherein the ratchet wheel has a toothed face facing toward the handwheel, and wherein the further end of the ratchet slider is located in one of grooves defined by the toothed face.

Additionally, the further end of the ratchet slider may define a beveled surface, wherein the toothed face defines chamfered surfaces between the grooves and teeth on opposite sides thereof, and wherein the ratchet slider is configured to control whether the ratchet wheel is rotated and the direction of rotation of the ratchet wheel through altering an angle of the beveled surface with respect to the chamfered surfaces.

Additionally, the beveled surface may be parallel to one of the chamfered surfaces so that the ratchet wheel is rotatable in one direction, or

Alternatively, the beveled surface may not be parallel to any of the chamfered surfaces so that the ratchet wheel is not rotatable.

Additionally, the retention mechanism may comprise a catheter holder, a clamp plate holder and a clamp plate, the catheter holder sleeved over a proximal end of the outer or inner tube, the clamp plate holder fixedly attached to the catheter holder so as to be oriented toward the synchronous conveying belt, the clamp plate retained on the clamp plate holder so that the synchronous conveying belt is clamped between the clamp plate and the clamp plate holder.

In a second aspect, the present invention provides an implant delivery system comprising the implant delivery handle as defined above and a catheter assembly, the catheter assembly comprising an outer tube and an inner tube, which are arranged one sleeved over the other from the outside inwards respectively, wherein the implant delivery handle drives the outer and inner tubes to move axially.

In a third aspect, the present invention provides an implant system comprising the implant delivery handle as defined above, an outer tube, an inner tube, a tapered tip and a retainer, the retainer disposed at a distal end of the inner tube, the tapered tip disposed at a distal end portion of the outer tube, the outer tube configured for an implant to be compressed within the distal portion of outer tube and located between the tapered tip and the retainer, wherein the implant delivery handle drives the outer and inner tubes to move axially to enable loading, delivery and release of the implant.

In a fourth aspect, the present invention provides a method of operation of the implant delivery system as defined above, which comprises:
pivoting the slider pin so that the beveled surface of the ratchet slider is not parallel to any chamfered surface of the toothed face and the ratchet slider is restricted in one of the grooves where the ratchet slider is located, and allowing safe self-locking of the inner or outer tube by the synchronous conveying device; and
pivoting the slider pin so that the beveled surface is parallel to one of the chamfered surfaces, making the ratchet wheel rotatable only in a first direction or a second direction opposite to the first direction and allowing transmission and guidance for the inner or outer tube by the synchronous conveying device,
wherein the proximal one of the conveyance sub-systems is coupled to the inner tube, and the distal one of the conveyance sub-systems is coupled to the outer tube.

Compared with the prior art, the present invention offers the benefits as follows:
It provides an implant delivery handle, an implant system, a delivery system and a method of operation thereof. The implant delivery handle comprises two conveyance sub-systems both oriented along an axial direction of a catheter assembly, in which a distal one is coupled to a proximal portion of an outer tube in the catheter assembly, and a proximal one is coupled to a proximal portion of an inner tube in the catheter assembly. Each of the conveyance sub-systems comprises a synchronous conveying device and a ratchet fixed to the synchronous conveying device. The ratchet device is configured to enable safe self-locking, transmission and guidance of the outer or inner tube through the synchronous conveying device. In this way, both the inner and outer tubes can be locked in one direction, achieving safe self-locking of the implant delivery handle. Uncontrolled displacement of the inner or outer tube can be avoided.

Further, the implant system, delivery system and method enable accurate operation of the implant delivery system and the delivery system and can prevent opposite movement of the inner or outer tube caused by incorrect operation of the operator, thereby avoiding uncontrolled displacement of the inner or outer tube.

### BRIEF DESCRIPTION OF THE DRAWINGS

Fig. 1 is a schematic perspective view of an implant delivery system according to an embodiment of the present invention.
Fig. 2 is a partial perspective view of an implant delivery handle according to an embodiment of the present invention.
Fig. 3 is a schematic enlarged view of part A of Fig. 1.
Fig. 4 is a schematic diagram illustrating the structure of a first retention mechanism according to an embodiment of the present invention.
Fig. 5 is a schematic perspective view of part of Fig. 2.
Figs. 6a to 6f schematically illustrate movement of a first ratchet wheel in cooperation with a first ratchet slider according to an embodiment of the present invention.

Description of Reference Numerals in Drawings:
1, catheter assembly; 110, inner tube; 120, outer tube; 130, guidewire tube; 140, tapered tip; 150, retainer;
2, implant delivery handle; 210, housing; 220, retaining rod; 3, implant;
300, first conveyance sub-system; 310, first retention mechanism; 311, first catheter holder; 312, first clamp plate holder; 313, first clamp plate; 320, first synchronous conveying device; 321, first driving pulley; 322, first driven pulley; 323, first synchronous conveying belt; 330, first handwheel; 340, first ratchet device; 341, first ratchet wheel; 3411, 3412, first chamfered surface; 3413, tooth; 3414, groove; 342, first ratchet slider; 3421, first beveled surface; 343, first slider pin; 344, first slider base; 3441, first body; 3442, first retainer; 345, first resilient member;
400, second conveyance sub-system; 410, second retention mechanism; 420, second synchronous conveying device; 421, second driving pulley; 422, second driven pulley; 423, second synchronous conveying belt; 430, second handwheel; and 440, second ratchet device.

### DETAILED DESCRIPTION

In principle, the present invention provides, in a first aspect thereof, an implant delivery handle comprising two conveyance sub-systems both oriented along an axis of a catheter assembly. A distal one of the conveyance sub-systems is coupled to a proximal portion of an outer tube in the catheter assembly, and a proximal one of the conveyance sub-systems is coupled to a proximal portion of an inner tube in the catheter assembly.

Each conveyance sub-system comprises a synchronous conveying device and a ratchet device fixed to the synchronous conveying device. The ratchet device is configured to enable safe self-locking, transmission and guidance of the outer or inner tube through the synchronous conveying device.

In a second aspect, there is provided an implant delivery system comprising the implant delivery handle and a catheter assembly comprising an outer tube and an inner tube, which are arranged one sleeved over the other from the outside inwards respectively. The implant delivery handle drives the outer and inner tubes to move axially.

In a third aspect, there is provided an implant system comprising the implant delivery handle, an outer tube, an inner tube, a tapered tip and a retainer. The retainer is disposed at a distal end of the inner tube, and the tapered tip is disposed at a distal end of outer tube. An implant is compressed within the distal portion of outer tube and located between the tapered tip and the retainer. The implant delivery handle drives the outer and inner tubes to move axially to enable loading, delivery and release of the implant.

In a fourth aspect, there is provided a method of operation of the implant delivery system, which comprises:
pivoting a slider pin so that a beveled surface of a ratchet slider is not parallel to any of chamfered surfaces defined by a toothed face, thereby restricting the ratchet slider in one of grooves where it is located and allowing safe self-locking of the inner or outer tube by a synchronous conveying device; and
pivoting the slider pin so that the beveled surface is parallel to one of the chamfered surfaces, thereby making a ratchet wheel rotatable only in a first direction or a second direction opposite to the first direction and allowing transmission and guidance for the inner or outer tube by the synchronous conveying device,
wherein the proximal conveyance sub-system is coupled to the inner tube, and the distal conveyance sub-system is coupled to the outer tube.

The implant delivery handle, implant system, delivery system and method of operation proposed in the present invention will be described in greater detail below. The invention will be described in greater detail below with reference to the accompanying drawings, which present preferred embodiments of the invention. It would be appreciated that those skilled in the art can make changes to the invention disclosed herein while still obtaining the beneficial results thereof. Therefore, the following description shall be construed as being intended to be widely known by those skilled in the art rather than as limiting the invention.

For the sake of clarity, not all features of actual implementations are described. In the following, description and details of well-known functions and structures are omitted to avoid unnecessarily obscuring the invention. It should be appreciated that in the development of any such actual implementation, numerous implementation-specific decisions must be made to achieve specific goals of the developers, such as compliance with system-related and business-related constrains, which may vary from one implementation to another. Moreover, it should be appreciated that such a development effort might be complex and time consuming, but would nevertheless be a routine undertaking for those of ordinary skill in the art.

Objects, advantages and features of the present invention will become more apparent upon reading the following detailed description of the present invention with reference to the accompanying drawings. Note that the figures are provided in a very simplified form not necessarily drawn to exact scale for the only purpose of facilitating easy and clear description of the embodiments disclosed herein. As used herein, the term "or" is generally employed in a sense including the meaning of "and/or", unless the context clearly dictates otherwise. As used herein, the terms "inner", "outer" and similar terms are merely illustrative and do not represent the only implementation possible. As used herein, the terms "proximal" and "distal" are employed to describe relative orientations, relative positions and directions between components of a medical device or actions thereof, as viewed by a surgeon operating the device. Without wishing to be limiting, a "proximal end" usually refers to an end closer to the operator, and a "distal end" usually refers to an end closer to the heart of a patient, during normal operation of the medical device.

The two conveyance sub-systems are referred to hereinafter respectively as a first conveyance sub-system and a second conveyance sub-system. They are synchronous conveying systems of substantially the same structure, except for slight differences, such as different synchronous conveying belt lengths and different dimensions of second and first catheter holders for retaining proximal ends of the inner and outer tubes, respectively, which, however, indeed, have no impact on functionality of the two sub-systems. Therefore, for the sake of brevity and clarity, only elements of the first conveyance sub-system are labeled in the annexed figures, and the first conveyance sub-system is described in greater detail than the second conveyance sub-system in the embodiments set forth below.

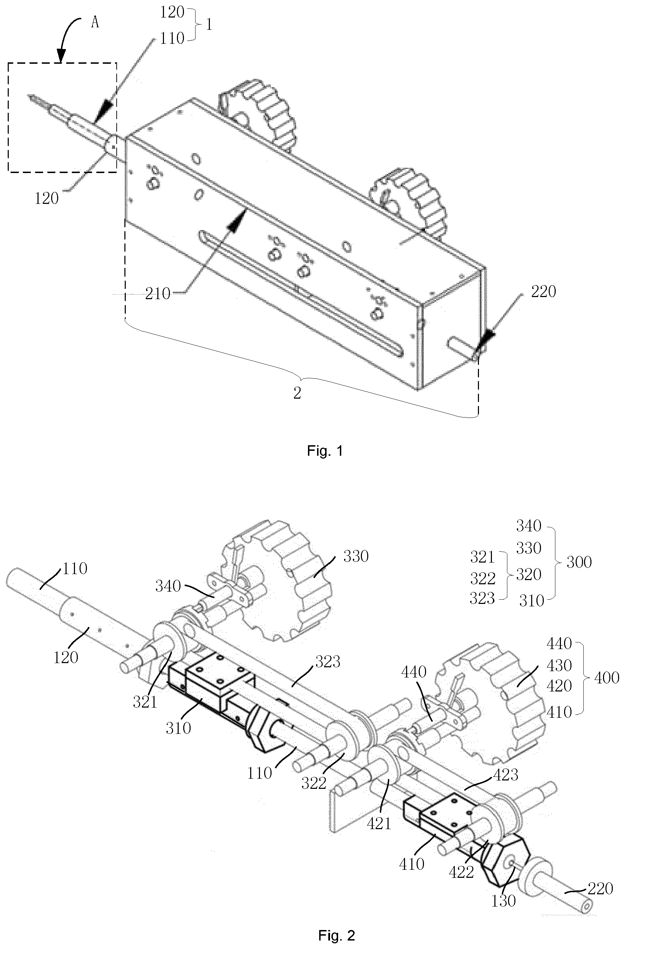

Fig. 1 is a schematic perspective view of an implant delivery system according to an embodiment of the present invention. As shown in Fig. 1, the implant delivery system according to the present embodiment includes a catheter assembly 1 and an implant delivery handle 2 for driving the outer and inner tubes to move axially.

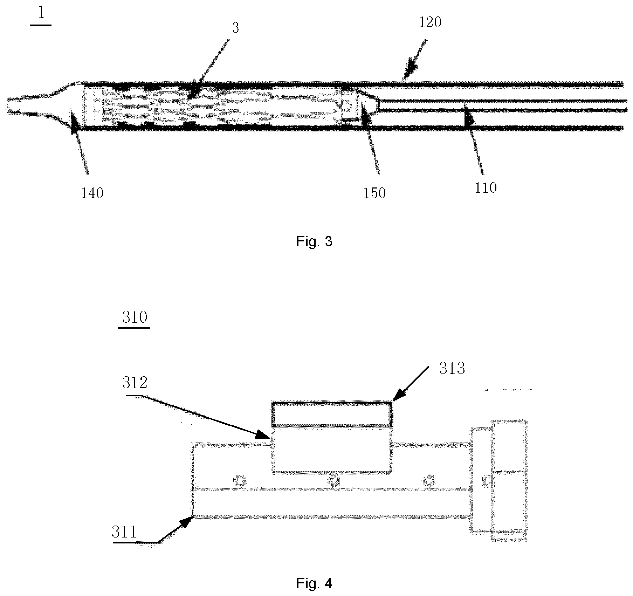

Fig. 2 is a partial perspective view of the implant delivery handle according to this embodiment, and Fig. 3 is an enlarged schematic view of part A of Fig. 1. As shown in Figs. 2 and 3, the catheter assembly 1 includes an outer tube 120, an inner tube 110 and a guidewire tube 130, which are arranged one sleeved over another from the outside inwards respectively. That is, the outer tube 120 is sleeved over the inner tube 110, and the inner tube 110 is sleeved over the guidewire tube 130. A guidewire is provided in the guidewire tube 130.

A distal portion of the catheter assembly 1 further includes a tapered tip 140 and a retainer 150. The retainer 150 is provided at a distal end of the inner tube 110 so as to be restricted in six degrees of freedom and thereby retain an implant 3. The tapered tip 140 is detachably provided at a distal end of the outer tube 120, and the implant 3 is disposed distal to the inner tube 110 and compressed within the distal portion of the outer tube 120. Before being released, the implant 3 is confined between the tapered tip 140 and the retainer 150. The implant 3 is a valve stent, for example.

With continued reference to Fig. 2, the implant delivery handle 2 includes a housing 210, a retaining rod 220, a first conveyance sub-system 300 and a second conveyance sub-system 400. The housing 210 is an enclosed rectangular parallelepiped assembly with distal and proximal sides each provided therein with a through hole. The through hole in the distal side is aligned with the through hole in the proximal side, and a straight line segment connecting the through holes in the distal and proximal sides defines an axial direction of the housing 210. The retaining rod 220 is distally secured in the through hole in the proximal side and coupled to a proximal portion of the catheter assembly 1. The catheter assembly 1 is inserted through the through hole in the distal side of the housing 210 and thus distally exposed outside of the housing 210. The catheter assembly 1 is coaxially with the housing 210. The first conveyance sub-system 300 and the second conveyance sub-system 400 are of the same structure. Both of them are partially disposed within, and hence protected by, the housing 210.

In the present embodiment, the distal portion of the retaining rod 220 retains a proximal portion of the guidewire tube 130, and the first conveyance sub-system 300 and the second conveyance sub-system 400 are partially housed in the housing 210 in the axial direction. The first conveyance sub-system 300 is located distally to the second conveyance sub-system 400. The second conveyance sub-system 400 is coupled to a proximal end of the inner tube 110 and configured to drive the inner tube 110 to axially move back and forth (axially from proximal to distal, or axially from distal to proximal) and to control transmission, guidance of the inner tube 110. The first conveyance sub-system 300 is coupled to a proximal end of the outer tube 120 and configured to drive the outer tube 120 to axially move back and forth and to control transmission, guidance of the outer tube 120.

The first conveyance sub-system 300 includes a first retention mechanism 310, a first synchronous conveying device 320, a first handwheel 330 and a first ratchet device 340. The first synchronous conveying device 320 includes a first driving pulley 321, a first driven pulley 322 and a first synchronous conveying belt 323 on both the first driving pulley 321 and the first driven pulley 322. The first driving pulley 321 is coupled to the first handwheel 330 such that the first handwheel 330 can drive the first driving pulley 321 to rotate, and the first driving pulley 321 in turn causes the first driven pulley 322 to rotate in a direction in which the first driving pulley 321 rotates, and hence the first synchronous conveying belt 323 to move forth and back in the axial direction of the housing 210. The first synchronous conveying belt 323 is disposed on one side of (e.g., above) the catheter assembly 1, and the first handwheel 330 is arranged outside of the housing 210.

Fig. 4 is a schematic diagram illustrating the structure of the first retention mechanism in this embodiment. As shown in Fig. 4, the first retention mechanism 310 is sleeved over a proximal end of the outer tube 120 and fixedly attached to the first synchronous conveying belt 323. The first retention mechanism 310 includes a first catheter holder 311, a first clamp plate holder 312 and a first clamp plate 313. The first catheter holder 311 is sleeved over the proximal end of the outer tube 120 and the first clamp plate holder 312 is fixedly attached to a side wall of the first catheter holder 311 so as to face toward the first synchronous conveying belt 323. The first clamp plate 313 is retained on the first clamp plate holder 312, with the first synchronous conveying belt 323 being clamped between the first clamp plate 313 and the first clamp plate holder 312. In this way, movement of the first synchronous conveying belt 323 will cause the first retention mechanism 310 to drive the outer tube 120 to axially move forth and back.

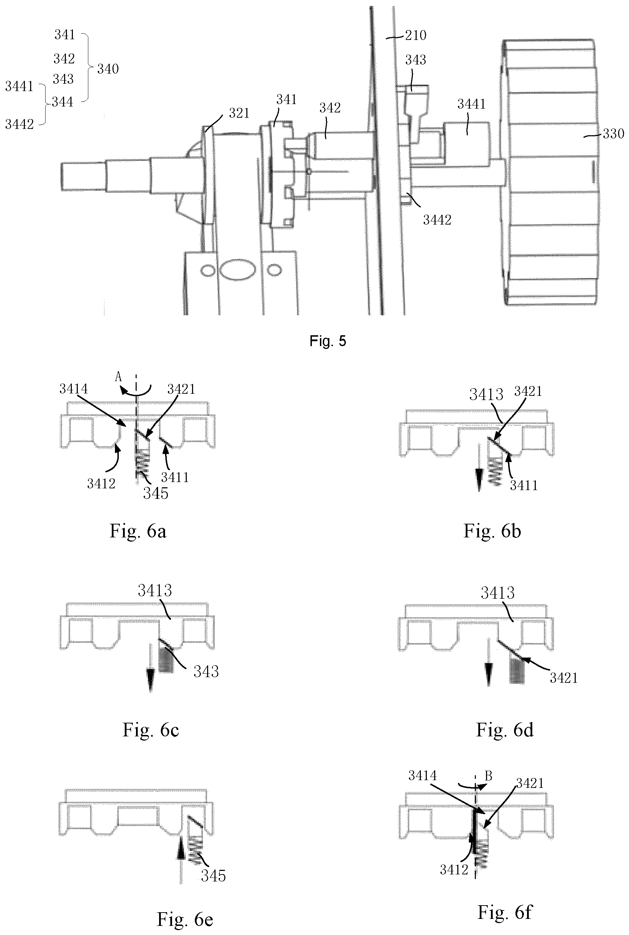

Fig. 5 is a schematic perspective view of part of Fig. 2b. Figs. 6a to 6f schematically illustrate movement of a first ratchet wheel in cooperation with a first ratchet slider in this embodiment. As shown in Figs. 5 to 6f, the first ratchet device 340 includes the first ratchet wheel 341, the first ratchet slider 342, a first slider pin 343 and a first slider base 344. The first ratchet wheel 341 is provided on the first driving pulley 321 so that the first driving pulley 321 can drive the first ratchet wheel 341 to rotate coaxially therewith. The first ratchet wheel 341 has an annular toothed face facing the first handwheel 330, and the annular toothed face has a center axis coinciding with a center axis of the first ratchet wheel 341. The toothed face defines teeth 3413 and grooves 3414 between adjacent teeth. Each groove 3414 defines first chamfered surfaces 3411, 3412 contiguous with the respective two adjacent teeth. The first chamfered surfaces 3411, 3412 are flat. One end of the first ratchet slider 342 is resiliently secured to the first slider base 344, and the other end is oriented toward the first ratchet wheel 341 and extends into one of the grooves 3414. The first slider base 344 is provided on an external wall of the housing 210.

The first slider base 344 includes a first body 3441 and a first retainer 3442. The first body 3441 is a hollow cylinder, which is open at one end and has a bottom portion opposite to the opening. The first ratchet slider 342 is received in, and resiliently coupled to, the first body 3441 so that it can resiliently move forth and back along an axis of the first body 3441. A first resilient member 345 is provided between the first body 3441 and an end portion of the first ratchet slider 342 closer to the first handwheel 330. The first resilient member 345 is provided on the bottom portion of the first body 3441 and can deform to drive the first ratchet slider 342 to resiliently move forth and back along the axis of the first body 3441. An end of the first ratchet slider 342 away from the first handwheel 330 defines a first beveled surface 3421 so that the first ratchet slider 342 has a right trapezoidal axial cross-section.

The first retainer 3442 is provided at the opening, and the first body 3441 defines a first cutout in one of its side walls closer to the opening, which allows the first slider pin 343 to be coupled to the first ratchet slider 342 in the first body 3441. The first body 3441 is retained on the housing 210 by the first retainer 3442. The first cutout is elongate in shape and defined in the circumference of the side wall. For example, it may extend half a circumferential length of the side wall of the first body 3441. This allows the first slider pin 343 to pivot in the first cutout from one side to the other, causing rotation of the first ratchet slider 342 in the first body 3441.

The first slider pin 343 is fixedly attached to one of the ends of the first ratchet slider 342 closer to the first handwheel 330 and disposed in the first cutout of the first body 3441. The first slider pin 343 can pivot in the first cutout to cause the first ratchet slider 342 to rotate, thus changing the orientation of the first beveled surface 3421.

As shown in Fig. 2, the second conveyance sub-system 400 is of the same structure as the first conveyance sub-system 300. In detail, the second conveyance sub-system 400 includes a second retention mechanism 410, a second synchronous conveying device 420, a second handwheel 430 and a second ratchet device 440. The second synchronous conveying device 420 includes a second driving pulley 421, a second driven pulley 422 and a second synchronous conveying belt 423 on both the second driving pulley 421 and the second driven pulley 422. The second driving pulley 421 is coupled to the second handwheel 430 such that the second handwheel 430 can drive the second driving pulley 421 to rotate, and the second driving pulley 421 in turn causes the second driven pulley 422 to rotate in a direction in which the second driving pulley 421 rotates, and hence the second synchronous conveying belt 423 to move forth and back in the axial direction of the housing 210. The second synchronous conveying belt 423 is disposed on one side of (e.g., above) the catheter assembly 1, and the second handwheel 430 is arranged outside of the housing 210.

The second retention mechanism 410 is sleeved over a proximal end of the inner tube 110 and fixedly attached to the second synchronous conveying belt 423. Since the second retention mechanism 410 is of the same structure as the first retention mechanism 310, for more details of this, reference can be made to the figure showing the first retention mechanism 310, i.e., Fig. 4. The second retention mechanism 410 includes a second catheter holder, a second clamp plate holder and a second clamp plate. The second catheter holder is sleeved over the proximal end of the inner tube 110 and the second clamp plate holder is fixedly attached to a side wall of the second catheter holder so as to face toward the second synchronous conveying belt. The second clamp plate is retained on the second clamp plate holder, with the second synchronous conveying belt being clamped between the second clamp plate and the second clamp plate holder. In this way, movement of the second synchronous conveying belt will cause the second retention mechanism to drive the inner tube 110 to axially move forth and back.

Likewise, the second ratchet device 440 is of the same structure as the first ratchet device 340. Referring to Figs. 3a to 3c, in conjunction with Fig. 2, the second ratchet device 440 includes a second ratchet wheel, a second ratchet slider, a second slider pin and a second slider base. The second ratchet wheel device 440 is provided on the second driving pulley 421 so that the second driving pulley 421 can drive the second ratchet wheel to rotate coaxially therewith. The second ratchet wheel has an annular toothed face facing the second handwheel 430, and the annular toothed face has a center axis in coincidence with a center axis of the second ratchet wheel. The toothed face defines teeth and grooves between adjacent teeth. Each groove defines second chamfered surfaces contiguous with the respective two adjacent teeth. The second chamfered surfaces are flat. One end of the second ratchet slider is resiliently secured to the second slider base, and the other end is oriented toward second ratchet wheel and extends into one of the grooves. The second slider base is provided on the external wall of the housing 210.

The second slider base includes a second body and a second retainer. The second body is of the same structure as the first body 3441, and the second ratchet slider is received in, and resiliently coupled to, the second body so that it can resiliently move forth and back along an axis of the second body. A second resilient member is provided between the second body and an end of the second ratchet slider closer to the second handwheel 430. The second resilient member is provided on the bottom portion of the second body and can deform to drive the second ratchet slider to resiliently move forth and back along the axis of the second body. An end portion of the second ratchet slider away from the second handwheel 430 defines a second beveled surface so that the second ratchet slider has a right trapezoidal axial cross-section.

The second retainer is provided at the opening, and the second body defines a second cutout in one of its side walls closer to the opening, which allows the second slider pin to be coupled to the second ratchet slider in the second body. The second body is retained on the housing 210 by the second retainer. The second cutout is elongate in shape and defined in the circumference of the side wall. For example, it may extend half a circumferential length of the side wall of the second body. This allows the second slider pin to pivot in the second cutout from one side to the other, causing rotation of the second ratchet slider in the second body.

The second slider pin is fixedly attached to one of the ends of the second ratchet slider around closer to the second handwheel 430 and disposed in the second cutout of the second body. The second slider pin can pivot in the second cutout to cause the second ratchet slider to rotate, thus changing the orientation of the second beveled surface.

Embodiments of the present invention also provide an implant system including the above-discussed implant delivery handle 2, an outer tube 120, an inner tube 110, a tapered tip 140 and a retainer 150. The retainer 150 is provided at a distal end of the inner tube 110, and the tapered tip 140 is provided at a distal end of the outer tube 120. An implant is compressed in the distal portion of the outer tube 120 and is located between the tapered tip 140 and the retainer 150. The implant delivery handle 2 can drive the outer tube 120 and the inner tube 110 to move axially to enable loading, delivery and release of the implant.

Embodiments of the present invention also provide a method of operation of an implant delivery system, which includes:
pivoting a slider pin so that a beveled surface is oriented at 90° with respect to chamfered surfaces, thereby restricting a ratchet slider in one of grooves where it is located and allowing safe self-locking of an inner or outer tube by a synchronous conveying device; and
pivoting the slider pin so that the beveled surface is oriented at 0° with respect to a chamfered surface, thereby making the ratchet wheel rotatable only in a first direction or a second direction opposite to the first direction and allowing transmission and guidance for the inner or outer tube by the synchronous conveying device,
wherein a proximal conveyance sub-system is coupled to the inner tube, and a distal conveyance sub-system is coupled to the outer tube.

In detail, during operation of the first ratchet device 340, as shown in Fig. 6a, the first slider pin 343 may be pivoted in the first cutout to alter the orientation of the first beveled surface 3421 so that it opposes and is parallel to one first chamfered surface 3411. At this time, as shown in Fig. 6b, the first ratchet wheel 341 may be rotated in a first direction A (to cause the first chamfered surface 3411 to approach the first beveled surface 3421). As shown in Figs. 6c to 6d, as the first ratchet wheel 341 is being rotated, the first chamfered surface 3411 and the tooth 3413 that defines it will approach, come into abutment with and push the opposing first beveled surface 3421. As a result, the first resilient member 345 will be compressed, and the first ratchet slider 342 will move toward the first handwheel 330. As shown in Fig. 6e, upon the first ratchet slider 342 sliding into the next groove, the first resilient member 345 will no longer be compressed, and the first ratchet slider 342 will be biased back to its initial position.

As shown in Fig. 6f, when the first ratchet wheel 341 is rotated in a second direction B, no compression force toward the first handwheel 330 will be generated between any first chamfered surface 3412 and the first ratchet slider 342. Therefore, the first ratchet slider 342 cannot move axially, and the first beveled surface 3421 will remain in a groove 3414 between adjacent two first chamfered surfaces 3411, 3412. The first direction A is opposite to the second direction B. As shown in Fig. 6f, when the first ratchet wheel 341 is rotated in the first direction A by manipulating the first handwheel 330, the first driving pulley 321 will drive the first synchronous conveying belt 323 and the outer tube 120 to advance or retract in the axial direction of the housing 210.

The first slider pin 343 may be pivoted in the first cutout to alter the orientation of the first beveled surface 3421 so that it is not parallel to any first chamfered surface 3411 or 3412. In this case, whether the first ratchet wheel 341 is rotated in the first direction A or in the second direction B, no compression force toward the first handwheel 330 will be generated between any first chamfered surface 3411 or 3412 and the first ratchet slider 342. Therefore, the first ratchet slider 342 cannot move axially, and the first beveled surface 3421 will remain in a groove 3414 between adjacent two first chamfered surfaces 3411, 3412. That is, the first ratchet device 340 is in a self-locking state.

During operation of the second ratchet device, the second slider pin may be pivoted in the second cutout to alter the orientation of the second beveled surface, so that it opposes and is parallel to one second chamfered surface. At this time, the second ratchet wheel may be rotated in a first direction, and as the second ratchet wheel is being rotated, the second chamfered surface and the tooth that defines it will approach, come into abutment with and push the opposing second beveled surface. As a result, the second resilient member will be compressed, and the second ratchet slider will move toward the second handwheel. Upon the second ratchet slider sliding into the next groove, the second ratchet slider will no longer be compressed, and the second resilient member will be biased back to its initial position.

When the second ratchet wheel is rotated in a second direction, no compression force toward the second handwheel will be generated between the second beveled surface of the second ratchet slider and the opposing second chamfered surface. Therefore, the second ratchet slider cannot move axially, and the second beveled surface will remain in the groove. The first direction is opposite to the second direction. When the second ratchet wheel is rotated in the first direction by manipulating the second handwheel, the second driving pulley will drive the second synchronous conveying belt and the inner tube to advance or retract in the axial direction of the housing.

The second slider pin may be pivoted in the second cutout to alter the orientation of the second beveled surface so that it is not parallel to either of the second chamfered surfaces on opposite sides of the groove where it is in. In this case, whether the second ratchet wheel is rotated in the first A or second B direction, no compression force toward the second handwheel 430 will be generated between either of the second chamfered surfaces and the second ratchet slider. Therefore, the second ratchet slider cannot move axially, and the second beveled surface will remain in the groove. That is, the second ratchet device 440 is in a self-locking state.

Use of the implant delivery handle may involve first pivoting the first slider pin 343 in the first cutout so that the first beveled surface 3421 is oriented proximally and becomes opposite and parallel to one first chamfered surface 3411. In this configuration, the first ratchet wheel 341 can be rotated only clockwise. Moreover, the second slider pin may be pivoted in the second cutout so that the second beveled surface is oriented proximally and becomes opposite and parallel to a second chamfered surface beside the groove where the end of the second ratchet slider away from the second handwheel is in. In this configuration, the second ratchet wheel can be rotated only clockwise. After that, the first handwheel 330 and the second handwheel 430 may be both rotated clockwise to cause the first conveyance sub-system 300 and the second conveyance sub-system 400, respectively, to drive the outer tube 120 and the inner tube 110, respectively, to move proximally. After the inner tube 110 and the outer tube 120 each proximally move a certain distance, the first slider pin 343 may be again pivoted in the first cutout so that the first beveled surface 3421 is not parallel to either of the first chamfered surfaces 3411, 3412, e.g., oriented at 90° with respect thereto, and the first ratchet slider 342 is therefore locked in the groove 3414. As a result, the first ratchet wheel 341 cannot be rotated either clockwise or counterclockwise. Moreover, the second ratchet slider may also be locked in the groove where it is in, making the second ratchet wheel unable to move either clockwise or counterclockwise. That is, both the first ratchet device 340 and the second ratchet device 440 are in a self-locking state. The tapered tip may be then removed, and an implant 3 may be loaded into the distal portion of the outer tube 120 and retained between the tapered tip 140 and the retainer. Afterward, the first slider pin 343 may be again pivoted in the first cutout to make the first ratchet wheel 341 rotatable only counterclockwise, and the second slider pin may be again pivoted in the second cutout to make the second ratchet wheel also rotatable only counterclockwise. Thereafter, the first handwheel 330 and the second hand wheel 340 may be both rotated counterclockwise to cause the first conveyance sub-system 300 and the second conveyance sub-system 400, respectively, to drive the outer tube 120 and the inner tube 110, respectively, to move distally. After they reach their predefined positions, the first ratchet device 340 and the second ratchet device 440 may be self-locked, followed by release of the implant 3. After that, the first slider pin 343 may be again pivoted in the first cutout to make the first ratchet wheel 341 rotatable only clockwise, and the second slider pin may be again pivoted in the second cutout to make the second ratchet wheel also rotatable only clockwise, thereby allowing proximal movement of the inner tube 110 and the outer tube 120. The release of the valve stent in this process may involve distal movement of the inner tube 110 and proximal movement of the outer tube 120, or distal or proximal movement of both the inner tube 110 and the outer tube 120.

In summary, the present invention provides an implant delivery handle, an implant system, a delivery system and a method of operation thereof. The implant delivery handle comprises two conveyance sub-systems both oriented along an axis of a catheter assembly, in which a distal one is coupled to a proximal portion of an outer tube in the catheter assembly, and a proximal one is coupled to a proximal portion of an inner tube in the catheter assembly. Each of the conveyance sub-systems comprises a synchronous conveying device and a ratchet device fixed to the synchronous conveying device. The ratchet device is configured to enable safe self-locking, transmission and guidance of the outer or inner tube through the synchronous conveying device. In this way, both the inner and outer tubes can be locked in one direction, achieving safe self-locking of the implant delivery handle. Uncontrolled displacement of the inner or outer tube can be avoided.

Further, the implant system, delivery system and method provided in the present invention enable accurate operation of the implant delivery system and the delivery system and can prevent opposite movement of the inner or outer tube caused by incorrect operation of the operator, thereby avoiding uncontrolled displacement of the inner or outer tube.

It is to be noted that, as used herein, the terms "first" and "second" are only meant to distinguish various components, elements, steps, etc. from each other rather than indicate logical or sequential orderings thereof, unless otherwise indicated or specified.

It is to be understood that while the invention has been described above with reference to preferred embodiments thereof, it is not limited to these embodiments. In light of the above teachings, any person familiar with the art may make many possible modifications and variations to the disclosed embodiments or adapt them into equivalent embodiments, without departing from the scope of the invention. Accordingly, it is intended that any and all simple variations, equivalent changes and modifications made to the foregoing embodiments based on the substantive disclosure of the invention without departing from the scope thereof fall within this scope.

## Claims

1. An implant delivery handle, comprising two conveyance sub-systems both oriented along an axial direction of a catheter assembly, wherein a distal one of the conveyance sub-systems is coupled to a proximal portion of an outer tube in the catheter assembly, and a proximal one of the conveyance sub-systems is coupled to a proximal portion of an inner tube in the catheter assembly,
wherein each of the conveyance sub-systems comprises a synchronous conveying device and a ratchet device fixed to the synchronous conveying device, the ratchet device configured to enable safe self-locking, transmission and guidance of the outer or inner tube through the synchronous conveying device.

2. The implant delivery handle of claim 1, wherein each of the conveyance sub-systems further comprises a retention mechanism and a handwheel, the retention mechanism coupled both to the synchronous conveying device and to the outer or inner tube, the handwheel coupled to the synchronous conveying device and configured to drive, through the synchronous conveying device and the retention mechanism, the outer or inner tube to move axially.

3. The implant delivery handle of claim 2, wherein the ratchet device comprises a ratchet wheel, a ratchet slider, a slider pin and a slider base,
the slider base configured to accommodate the ratchet slider;
the ratchet slider having one end resiliently secured to the slider base and a further end disposed near the ratchet wheel, the ratchet slider configured to control whether the ratchet wheel is rotated and a direction of rotation of the ratchet wheel;
the ratchet wheel fixed to the synchronous conveying device and configured to cooperate with the ratchet slider to control whether the outer or inner tube to move axially and a direction of axial movement of the outer or inner tube,
the slider pin coupled to the one end of the ratchet slider and configured to drive the ratchet slider to move.

4. The implant delivery handle of claim 3, wherein the synchronous conveying device comprises a driving pulley, a driven pulley and a synchronous conveying belt, the synchronous conveying belt provided on both the driving pulley and the driven pulley, the driving pulley disposed coaxially with the handwheel so as to rotate therewith, the driving pulley configured to drive the driven pulley to rotate in a direction in which the driving pulley rotates and cause the synchronous conveying belt to move in an axial direction of the outer tube.

5. The implant delivery handle of claim 4, wherein the ratchet wheel is disposed coaxially with the handwheel and is located between the driving pulley and the handwheel, wherein the ratchet wheel has a toothed face facing toward the handwheel, and wherein the further end of the ratchet slider is located in one of grooves defined by the toothed face.

6. The implant delivery handle of claim 5, wherein the further end of the ratchet slider defines a beveled surface, wherein the toothed face defines chamfered surfaces between the grooves and teeth on opposite sides thereof, and wherein the ratchet slider is configured to control whether the ratchet wheel is rotated and the direction of rotation of the ratchet wheel through altering an angle of the beveled surface with respect to the chamfered surfaces.

7. The implant delivery handle of claim 6, wherein the beveled surface is parallel to one of the chamfered surfaces so that the ratchet wheel is rotatable in one direction, or
wherein the beveled surface is not parallel to any of the chamfered surfaces so that the ratchet wheel is not rotatable.

8. The implant delivery handle of claim 3, wherein the retention mechanism comprises a catheter holder, a clamp plate holder and a clamp plate, the catheter holder sleeved over a proximal end of the outer or inner tube, the clamp plate holder fixedly attached to the catheter holder so as to be oriented toward the synchronous conveying belt, the clamp plate retained on the clamp plate holder so that the synchronous conveying belt is clamped between the clamp plate and the clamp plate holder.

9. An implant delivery system, comprising the implant delivery handle of any one of claims 1 to 8 and a catheter assembly, the catheter assembly comprising an outer tube and an inner tube, which are arranged one sleeved over the other from the outside inwards respectively, wherein the implant delivery handle drives the outer and inner tubes to move axially.

10. An implant system, comprising the implant delivery handle of any one of claims 1 to 8, an outer tube, an inner tube, a tapered tip and a retainer, the retainer disposed at a distal end of the inner tube, the tapered tip disposed at a distal end of the outer tube, the outer tube configured for an implant to be compressed within a distal portion of the outer tube and located between the tapered tip and the retainer, wherein the implant delivery handle drives the outer and inner tubes to move axially to enable loading, delivery and release of the implant.

11. A method of operation of the implant delivery system of claim 9, comprising:
pivoting a slider pin so that a beveled surface of a ratchet slider is not parallel to a chamfered surface of a toothed face and the ratchet slider is restricted in a groove where the ratchet slider is located, and allowing safe self-locking of the inner or outer tube by the synchronous conveying device; and
pivoting the slider pin so that the beveled surface is parallel to the chamfered surface, making the ratchet wheel rotatable only in a first direction or a second direction opposite to the first direction, and allowing transmission and guidance for the inner or outer tube by the synchronous conveying device,
wherein the proximal one of the conveyance sub-systems is coupled to the inner tube, and the distal one of the conveyance sub-systems is coupled to the outer tube.
